**Europäisches Patentamt**

**European Patent Office**

**Office européen des brevets**

(19)

(11) Veröffentlichungsnummer: **0 161 663**
A1

(12) ## EUROPÄISCHE PATENTANMELDUNG

(21) Anmeldenummer: **85105897.4**

(22) Anmeldetag: **14.05.85**

(51) Int. Cl.⁴: **A 61 F 13/16,** A 61 F 13/18

(30) Priorität: **15.05.84 DE 3417910**

(43) Veröffentlichungstag der Anmeldung: **21.11.85**
**Patentblatt 85/47**

(84) Benannte Vertragsstaaten: **AT BE CH DE FR GB IT LI LU NL SE**

(71) Anmelder: **Dobson, Brigitte, Seeadlerring 3, D-3004 Isernhagen 1 (DE)**

(72) Erfinder: **Dobson, Brigitte, Seeadlerring 3, D-3004 Isernhagen 1 (DE)**

(54) **Neuartige hygienische Vorlage.**

(57) Beschrieben wird eine neuartige, hygienische Vorlage, die zwischen den Labia minora getragen werden kann.

EP 0 161 663 A1

0161663

Neuartige hygienische Vorlage

Die Erfindung betrifft eine hygienische Vorlage, die die
Ausscheidungen und Sekretionen beispielsweise während der
Menstruation auffängt.

Als Vorlagen sind beispielsweise Damenbinden und Slipeinlagen
bekannt, die zum Schutz der Kleidung die Menstruationsblutungen und andere Sekretionen auffangen. Entscheidend für
diese Schutzwirkung ist eine exakte und dauerhafte Fixierung
dieser Vorlagen. Lösungsvorschläge finden sich in Form von
Haltegürteln, speziell gestalteten Monatshöschen oder
neuerdings in Binden, die mit Klebestreifen zur Befestigung im
Slip versehen sind. All diese Lösungen bieten jedoch nur einen
mehr oder weniger genügenden Schutz der Kleidung. Die Gründe
hierfür liegen darin, daß sich die Binden beim Gehen, durch
Veränderung der Körperstellung und durch Anpassung an den
Körper meist automatisch zusammendrücken oder so verrutschen,
daß die Ausscheidungen an den Seiten vorbeifließen können.

Ein zusätzlich auftretendes Problem besteht darin, daß zu
Beginn der Regel oder bei starken Blutungen für die meisten
Frauen intralabial ein unangenehmes Gefühl auftritt, ein
Problem, das mit keiner der bekannten Binden gelöst wird.

Abhilfe bieten hier zwar die bekannten Tampons, doch saugen
diese nicht nur die Menstruationssekrete, sondern auch die
natürliche Scheidenflüssigkeit auf, so daß die Mucosa
austrocknen kann. Rötungen, Entzündungen und die Gefahr
vermehrten Pilzbefalls können die Folge sein. Nicht zuletzt
stehen einige Frauen aus persönlichen Gründen der Anwendung
von Tampons mit gewisser Abneigung gegenüber.

Die US-PS 29 17 049 beschreibt eine hygienische Vorrichtung,
die die Form eines dreieckigen, kegelförmigen Saugkörpers aufweist. Diese Vorrichtung wird mit der breiten Basis so
zwischen die Labia minora geschoben, daß sich der größte Teil
der Vorrichtung oberhalb der Labia minora, also quasi
innerhalb des Körpers befindet, und nur die als nicht
saugfähige feste Griffkante ausgestaltete Schmalseite der
Vorrichtung sich zwischen der Labia minora befindet. Der
eigentliche Saugkörper befindet sich also bei Gebrauch,
ähnlich wie bei einem Tampon, innerhalb des Körpers, doch
anders als bei diesen an einer Stelle der Vulva, die äußerst
sensibel und reizempfindlich ist; wenn überhaupt so dürfte
diese Vorrichtung wohl nur in wenigen Ausnahmesituationen
"ertragbar" sein. Keinesfalls erscheint sie jedoch dazu
geeignet sie beispielsweise während der normalen Menstruation
zu tragen.

Aufgabe der vorliegenden Erfindung war es daher, eine Vorlage
zu entwickeln, die
  a) beim Gehen oder durch Veränderung der Körperstellung
   ʼ nicht verrutscht,
  b) in wesentlichen Teilen bei der Benutzung nicht
     zusammengedrückt wird und
  c) das unangenehme intralabiale Gefühl bei starkem Ausfluß
     verhindert.

Erfindungsgemäß wird diese Aufgabe durch eine Vorlage gelöst,
die einen Teil aufweist, der in Abmessung und Form den
Innenflächen der Labia minora anatomisch angepaßt ist und
zwischen diesen getragen wird, während der Rest als
Hauptsaugkörper sich außerhalb der Labia minora befindet.
Dieser intralabiale Teil kann als eigenständige Vorlage oder
als Teil einer Vorlage ausgebildet sein.

Hergestellt werden kann die erfindungsgemäße Vorlage aus einem
blut- und sekretdurchlässigen Gewebe, das ein blut- und
sekretaufnehmendes Material umgibt. Das saugfähige Material
kann beispielsweise eine mit Quellmitteln versehene Watte

0161663

sein, die zur Formgebung aus nicht gewebtem Material, beispielsweise in Form einer Membrane umgeben ist. Dadurch ist es möglich, die in weiten Grenzen dem Verwendungszweck entsprechende Form individuell anzupassen.

Eine mögliche Form des intralabialen Teils ist in Abb.I dargestellt (der Teil, der bei Gebrauch dem Körper zugewandt ist, ist in den Abbildungen I-III oben dargestellt). Sie besteht aus einer Basis, die rechteckig geformt sein kann. Ebenso können die Schmal- und/oder die Längsseiten elipsoid geformt sein (Abb.IV). Abb.II zeigt einen Schnitt durch die latero-medial Achse mit gebogenen, sich in einem Punkt treffenden Seiten. Diese Seiten können auch gerade sein und/oder eine weitere Seite einschließen, die ebenfalls gerade oder gebogen sein kann, so daß entweder ein keilförmiger oder ein trapezoider Körper mit geraden und/oder gebogenen Seiten entsteht. Der Längsschnitt parallel zur antero-posteriorAchse (Abb.III) zeigt von b auf d eine ansteigende Kurve und von d auf c eine abfallende Kurve. Dieser Kurvenverlauf ist auch als Halbkreis oder in einer der Anatomie des Perineums angepassten Form und der Punkt d als Fläche auszubilden.

Der im Querschnitt Abb.II mit a gekennzeichnete Hohlraum ist mit einem flüssigkeits- und blutresorbierendem Material auszufüllen. Vorzugsweise ist dieses weich und flexibel, damit es sich möglichst genau den Konturen der Innenflächen der Labia minora anpassen kann. Schicht e in Abb.II stellt eine Kunststoffschicht, vorzugsweise aus Polyvinylalkohol dar, die wahlweise eingebracht werden kann. Ebenso gilt dies für den mit f bezeichneten Klebestreifen.

Der intralabiale Teil kann beispielsweise für sich allein verwendet oder mit einer Binde, Slipeinlage oder Vorlage kombiniert werden, wobei die Teile auch beispielsweise durch Klebe- oder Klettvorrichtungen oder durch Nähte befestigt werden können. Weiterhin ist es möglich, die erfindungsgemäße Vorlage an der perinealen Fläche im Bereich der Labia mit
einer lockeren, übergroßen Membrane zu versehen, die das
resorbierende Material umgibt, so daß das intralabiale Teil
durch Zupfen an dem resorbierenden Material individuell vor
der Verwendung gemäß der Erfindung geformt werden kann.

Um ein sicheres und bequemes Tragen zu ermöglichen, kann eine
schmale, gegebenenfalls weiche Verlängerung im hinteren
Bereich der Vorlage vorgesehen werden, die sich dem glutealen
Spalt anpassen kann. Aus dem gleichen Grund kann auch der
vordere Bereich der Vorlage eine Verlängerung aufweisen.

Die Abbildungen geben die Abmessungen der erfindungsgemäßen
Vorlage ungefähr im Maßstab 1:1 wieder. Ebenso sind jedoch
auch andere Abmessungen möglich.

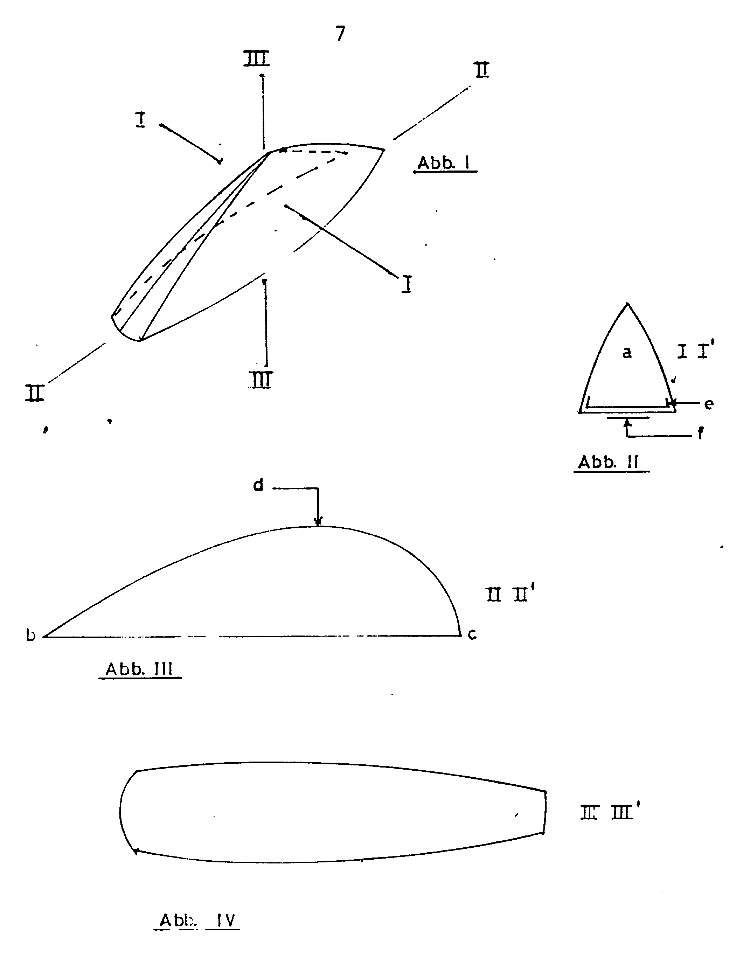

PATENTANSPRÜCHE

1) Vorlage, die Ausscheidungen und Sekretionen auffängt, dadurch gekennzeichnet, daß zumindest ein Teil der inneren Form der Labia minora anatomisch angepasst und zwischen diesen zu tragen ist.

2) Vorlage nach Anspruch 1, dadurch gekennzeichnet, daß der Schnitt durch die latero-medial-Achse des intralabialen Teils einen keilförmig ausgebildeten Körper mit geraden und/oder gebogenen Seiten zeigt.

3) Vorlage nach Anspruch 1, dadurch gekennzeichnet, daß der Schnitt durch die latero-medial-Achse des intralabialen Teils einen trapezoiden Körper mit geraden und/oder gebogenen Seiten zeigt.

4) Vorlage nach einem der vorhergehenden Ansprüche, dadurch gekennzeichnet, daß die obere, perineale Grenze in dem Längsschnitt parallel zur antero-posterior-Achse des intralabialen Teils einen Bogen beschreibt, der eine Ebene einschließt.

5) Vorlage nach einem der vorhergehenden Ansprüche, dadurch gekennzeichnet, daß sie aus einem blut- und sekretdurchlässigen Gewebe besteht, das mit blut- und sekretaufnehmendem Material gefüllt ist.

6) Vorlage nach einem der vorhergehenden Ansprüche, dadurch gekennzeichnet, daß sie aus weichen, formbaren Materialien hergestellt ist.

7) Vorlage nach einem der vorhergehenden Ansprüche, dadurch gekennzeichnet, daß sie vor Verwendung individuell verformbar ist.

0161663

8) Vorlage nach einem der vorhergehenden Ansprüche, dadurch gekennzeichnet, daß sie an der perinealen Fläche im Bereich der Labia minora mit einem lockeren, übergrossen, das resorbierende Material umgebende Gewebe versehen ist, um durch Verformung des blut- und sekretaufnehmenden Materials ein individuell angepaßtes intralabiales Teil der Vorlage erhalten zu können.

9) Vorlage nach einem der vorhergehenden Ansprüche, dadurch gekennzeichnet, daß die Vorlage im hinteren Bereich eine schmale Verlängerung aufweist, die sich dem glutealen Spalt anpassen kann.

10) Vorlage nach einem der vorhergehenden Ansprüche, dadurch gekennzeichnet, daß die Vorlage im vorderen Bereich eine Verlängerung aufweist.

11,) Vorlage nach einem der vorhergehenden Ansprüche, dadurch gekennzeichnet, daß zwischen dem äußeren, durchlässigen Gewebe und dem blut- und sekretaufnehmendem Material inferior ein aus einem geeigneten Kunststoff hergestellter Film eingearbeitet ist.

12) Vorlage nach Anspruch 11, dadurch gekennzeichnet, daß der eingearbeitete Film aus Polyvinylalkohol hergestellt ist.

13) Vorlage nach einem der vorhergehenden Ansprüche, dadurch gekennzeichnet, daß der extralabiale Teil mit einem Klebestreifen versehen ist.

1(1

0161663

Abb. I

Abb. II

Abb. III

Abb. IV

Europäisches
Patentamt

**EUROPÄISCHER RECHERCHENBERICHT**

| | EINSCHLÄGIGE DOKUMENTE | | EP 85105897.4 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US - A - 4 433 972 (MALFITANO)<br><br>* Ansprüche 1,3; Spalte 1, Zeile 48 - Spalte 2, Zeile 6; Spalte 2, Zeilen 13-15; Fig. * | 1-6 | A 61 F 13/16<br>A 61 F 13/18 |
| X | US - A - 3 905 372 (DENKINGER)<br><br>* Anspruch 1; Spalte 1, Zeile 65 - Spalte 2, Zeile 2; Spalte 2, Zeile 59 - Spalte 3, Zeile 2; Fig. 3-7 * | 1-10 | |
| X | US - A - 2 662 527 (JACKS)<br><br>* Anspruch 1; Fig. 1,5; Spalte 2, Zeilen 31-35, 47-50 * | 1 | |
| A | US - A - 3 528 422 (HODAS)<br><br>* Anspruch 1; Fig. 9 * | 1,5-10 | RECHERCHIERTE SACHGEBIETE (Int. Cl.4)<br><br>A 61 F |
| A | DE - A1 - 3 015 538 (ZENMI)<br><br>* Anspruch 1 * | 1,11, 12 | |
| A | DE - A1 - 2 516 220 (PERSONAL PRODUCTS)<br><br>* Anspruch 1 * | 1,13 | |

Der vorliegende Recherchenbericht wurde für alle Patentansprüche erstellt.

| Recherchenort | Abschlußdatum der Recherche | Prüfer |
|---|---|---|
| WIEN | 25-07-1985 | FARNIOK |

**EUROPÄISCHER RECHERCHENBERICHT**

Europäisches
Patentamt

## EINSCHLÄGIGE DOKUMENTE

EP 85105897.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 136 014 (SCHICKEDANZ) <br> * Fig. 1,2 * <br> -- | 1,9 | |
| A | FR - A1 - 2 420 339 (BEGHIN-SAY) <br> * Anspruch 1 * <br> ---- | 1 | |

RECHERCHIERTE
SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| WIEN | 25-07-1985 | FARNIOK |